(19) Europäisches Patentamt
European Patent Office
Office européen des brevets

(11) **EP 4 272 640 A1**

(12) **EUROPEAN PATENT APPLICATION**

(43) Date of publication:
**08.11.2023 Bulletin 2023/45**

(21) Application number: **22172071.7**

(22) Date of filing: **06.05.2022**

(51) International Patent Classification (IPC):
**A61B 5/11** (2006.01)     **A61B 5/00** (2006.01)

(52) Cooperative Patent Classification (CPC):
**A61B 5/11; A61B 5/6829;** A61B 2560/0443

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Haute Ecole du Paysage, d'Ingénierie et d'Architecture de Genève (Hépia)**
**1202 Geneva (CH)**

(72) Inventors:
• **PRAZ, Quentin**
**1202 Geneva (CH)**

• **PASSERAUB, Philippe**
**1202 Geneva (CH)**
• **SCHOINAS, Spyridon**
**1202 Geneva (CH)**
• **ASSAL, Mathieu**
**1206 Geneva (CH)**
• **ACKER, Antoine**
**1206 Geneva (CH)**
• **RAYMOND, Nils**
**1206 Geneva (CH)**

(74) Representative: **KATZAROV S.A.**
**Geneva Business Center**
**12 Avenue des Morgines**
**1213 Petit-Lancy (CH)**

(54) **EVALUATION OF THE FOREFOOT RELATIVE MOBILITY**

(57)     The present relates to a device for evaluating the relative mobility of a forefoot of a patient, the device comprising an actuator, a transmission element and two output shafts namely a first output shaft and a second output shaft,

the transmission element being configured for equally distributing an axial force provided by the actuator along the sagittal axis of the patient to the first output shaft and to the second output shaft.

The device further comprises a measuring module comprising a force sensor configured for measuring the axial force exerted by the actuator, and a displacement sensor configured for measuring the relative displacement of the first metatarsal head M1 relative to the lesser metatarsal heads M2-M5 along said sagittal axis.

The measuring module is configured for correlating said displacement measurements with said measured axial forces so as to evaluate the relative mobility of the forefoot of the patient.

Figure 1

# Description

## Technical Field

The present disclosure relates to a device and a system comprising said device for evaluating the relative mobility of a forefoot of a patient.

The present invention also relates to a method, preferably a computer implemented method, for computing a relative mobility score of a forefoot of a patient.

## Background of the art

The issue of a non-objective method in the examination of the forefoot relative mobility inspired numerous researchers to develop various methods and instruments. In the 1990s, Klaue and Glasoe were the first to propose and validate a technical solution that was able to quantify the first ray mobility in dorsiflexion. These devices are performing a relative displacement measurement with the foot in a neutral position while an automatized fixed force or manual unquantified force was only applied under the first metatarsal head. From this, Klaue estimated the threshold of forefoot mobility to be 8 millimeters or more by conducting studies with feet with Hallux Valgus and without. This study revealed a higher mobility for feet with Hallux Valgus.

Based on these pioneered methods and instruments, various other solutions conceived to propose a simpler measuring system that would be closer to the clinical examination. Nevertheless, a "simpler device" does not mean a device relevant for making appropriate therapeutic decisions from the clinical assessment, mainly due to the lack of reliable data generated induced by a great inter-observer variability.

For instance, a plastic instrument has been developed and consists of two millimetric scale pieces placed above the first metatarsal head accompanying the manual examination. It has the advantage of being low-cost and easy to use for daily practice. However the amount of applied force from the practitioner is not standardized, which makes the instability hard to assess. It is also impossible to provide objective monitoring of the pathology.

Other more complex solutions associated the gesture of the practitioner with an optical measurement system that recorded by a camera the angular deformation in dorsiflexion of the first metatarsophalangeal joint while the practitioner applies a force on the first and lesser metatarsal heads to measure a millimetric distance.

The majority of these solutions were used only for medical research purposes due to their numerous disadvantages, such as their lack of reliability, validity or responsiveness, not being practical for daily clinical use and being too complex. In addition, the existing instruments do not allow the assessment of instability reflecting a stance phase, where all metatarsals are loaded by the ground reaction force, which is essential for clinical decision making. None of these studies was able to propose forefoot instability classification correlating the assessment to the clinical decision.

Therefore, there is a need to provide an improved approach for measuring relative mobility of the forefoot of a patient to overcome or limit the drawbacks of the existing instruments.

## Summary of the invention

The above problems are solved or at least minimized by the present invention.

The invention concerns a device for evaluating the relative mobility of a forefoot of a patient, the device comprising an actuator, a transmission element and two output shafts namely a first output shaft and a second output shaft,

the transmission element being configured for equally distributing an axial force provided by the actuator along the sagittal axis of the patient to the first output shaft and to the second output shaft, the first output shaft being configured for bearing against the first metatarsal head M1 of the forefoot whereas the second output shaft being configured for bearing against the lesser metatarsal heads M2-M5 of the forefoot,

characterized in that the device further comprises a measuring module comprising

a force sensor configured for measuring the axial force exerted by the actuator, and

a displacement sensor configured for measuring the relative displacement of the first metatarsal head M1 relative to the lesser metatarsal heads M2-M5 along said sagittal axis when beard respectively against the first output shaft and on the second output shaft,

the measuring module being configured for correlating said displacement measurements with said measured axial forces so as to evaluate the relative mobility of the forefoot of the patient.

In another aspect, the invention concerns a system for evaluating the relative mobility of a forefoot of a patient, the system comprising

a device according to the present invention;

an orthosis configured for maintaining the forefoot of the patient in position suitable for measuring the relative mobility between the first metatarsal head and the lesser metatarsal heads of a forefoot of a patient;

a positioning module configured for reversibly fastening the device to the orthosis.

In another aspect, the invention relates to a method, preferably computer implemented method, for computing a relative mobility score a forefoot of a patient, the method comprising

i) providing a set of data (xi,yi) where

xi represents a measured applied force along the sagittal axis and equally distributed on the first metatarsal head M1 of the forefoot and on the lesser metatarsal heads M2-M5 of the forefoot, and

yi represents a relative displacement of the first metatarsal head M1 relative to the lesser metatarsal heads M2-M5 along said sagittal axis upon application of the corresponding measured xi applied force,

ii) representing the relative displacement of the first metatarsal head M1 relative to the lesser metatarsal heads M2-M5 (yi) as a function of the measured applied force (xi) for each (xi,yi);

iii) determining Xmin and Xmax as respectively the minimum measured force applied and the maximum measured force applied, and
determining Ymin and Ymax as respectively the minimum measured relative displacement for Xmin and the maximum measured relative displacement at Xmax,

iv) computing the relative mobility score RM as

$$RM = Ymax - Ymin.$$

In another aspect, the invention relates to a method, preferably computer implemented method, for computing a differential stiffness score a forefoot of a patient, the method comprising

i) providing a set of data (xi,yi) where

xi represents a measured applied force along the sagittal axis and equally distributed on the first metatarsal head M1 of the forefoot and on the lesser metatarsal heads M2-M5 of the forefoot, and

yi represents a relative displacement of the first metatarsal head M1 relative to the lesser metatarsal heads M2-M5 along said sagittal axis upon application of the corresponding measured xi applied force,

ii) representing the relative displacement of the first metatarsal head M1 relative to the lesser metatarsal heads M2-M5 (yi) as a function of the measured applied force (xi) for each (xi,yi);

iii) determining Xmin and Xmax as respectively the minimum measured force applied and the maximum measured force applied, and
determining Ymin and Ymax as respectively the minimum measured relative displacement for Xmin and the maximum measured relative displacement at Xmax,

iv) calculating the local derivatives at any points of the curve representing the relative displacement as a function of the force applied between Xmin, Xmax and Ymin, Ymax.

v) computing the differential stiffness score as the maximum value of the calculated local derivatives.

**Description of the invention**

The invention concerns a device for evaluating the relative mobility of a forefoot of a patient, the device comprising an actuator, a transmission element and two output shafts namely a first output shaft and a second output shaft,

the transmission element being configured for equally distributing an axial force provided by the actuator along the sagittal axis of the patient to the first output shaft and to the second output shaft, the first output shaft being configured for bearing against the first metatarsal head M1 of the forefoot whereas the second output shaft being configured for bearing against the lesser metatarsal heads M2-M5 of the forefoot,

characterized in that the device further comprises a measuring module comprising

a force sensor configured for measuring the axial force exerted by the actuator, and

a displacement sensor configured for measuring the relative displacement of the first metatarsal head M1 relative to the lesser metatarsal heads M2-M5 along said sagittal axis when beard respectively against the first output shaft and on the second output shaft,

the measuring module being configured for correlating said displacement measurements with said measured axial forces so as to evaluate the relative mobility of the forefoot of the patient.

Advantageously, the present invention allows to evaluate, preferably to quantify, the relative mobility as a function of the force applied between the first metatarsal head and the lesser metatarsal heads under equal force load. This is done by measuring the applied force and the resulting displacement corresponding to said measured applied force.

The functioning of the present invention advantageously replicates or mimics the orthopedic surgeon's gesture during a manual examination of patient forefoot to evaluate the relative stability by manual technics.

In contrast to the other existing methods, the proposed solution is biomechanically representative of a progressive weight-bearing phase of the gait.

Advantageously, the present invention allows to assist practitioners involved in the forefoot treatment, who want to make the most appropriate therapeutic decision for each patient's pathology.

The present invention offers data-driven biomechanical measurements of the forefoot's mobility and ensures objectivity with standardized measurement. It is possible to measure the force applied on the forefoot so that a given load can be reproduced by another practitioner to the one that performs the measurement in the first place. In other words, the present invention rationalizes the measuring of the relevant forefoot parameters (in particular displacement and the force applied) to avoid or at least limit the inter-observer variability. Therefore, the present invention can be considered as an objective method to evaluate the relative mobility of a patient's forefoot.

The present invention allows to personalize the forefoot analysis to each patient by applying the required force to obtain a relevant displacement measurement.

Preferably, the axial force applied by the actuator on the forefoot is exerted along the sagittal axis, more preferably in the dorsal direction.

Preferably, the relative displacement of the first metatarsal head M1 relative to the lesser metatarsal heads M2-M5 is measured along the sagittal axis, more preferably in the dorsal direction.

In a preferred embodiment, the measuring module is configured for measuring simultaneously the force applied and the relative displacement. Thus, each measured displacement corresponds to a measured applied force. This allows to associate to a measured applied force a corresponding displacement measurement. Preferably, the measurement of the relative displacement corresponding to an applied force are measured simultaneously, meaning with an interval less than or equal to about 1 ms.

Preferably, the device is configured for evaluating the relative mobility of the patient when the patient is in a standing position and in a lying position and any positions between a standing position and a lying position. In other words, the present invention can be used in multiple configurations, namely with a patient in a standing up position or in a lying position or in another position in between.

The lying position corresponds to the position of a patient examined by a practitioner.

When the device (or system) is used on a patient in a seated position (as an example of a position between a lying position and a standing position, the patient simulates the load on the forefoot of a standing position during evaluation, but with less force applied on said forefoot, meaning a partial weight bearing, for instance about 40% less. For instance, the vertical force of the lower limb is approximately 10% of the total body weight.

When the device (or system) is used on a patient in a standing position, the patient simulates the maximum static load on the forefoot during evaluation.

In a preferred embodiment the actuator is configured for being actuated manually by a user of the device providing the axial force, preferably the actuator comprising a hand to be handled by the user. For instance, the actuator is handled by the practitioner taking the measurements of a forefoot of a patient to evaluate the relative mobility of said forefoot. The force applied on the forefoot is transmitted by the practitioner to the forefoot via the actuator. Advantageously, a manual actuator provides a simple and inexpensive device with a small footprint, notably a low energy consumption and a low $CO_2$ footprint.

Preferably, the actuator is configured for being actuated by driving element commanding the actuation, preferably a motor, to provide the axial force. The present invention can be driven by a motorized element providing the applied force exerted on the forefoot of the patient. This allows reproducible dynamic measurements. It also reduces the influence of the user on the measurements.

In a preferred embodiment the device further comprises a computation unit configured for processing the measurements acquired by the measuring module to evaluate the relative mobility as a function of the measured axial force with respect to the measured relative displacement.

Preferably, the computation unit is embedded in the housing of the device. For instance, the computation unit comprises a processing unit, such as a microcontroller configured for processing the measured displacements and the measured applied forces to evaluate the relative mobility as a function of the measured axial force with respect to the measured relative displacement. In this embodiment, the device is independent, i.e. autonomous, from any other calculation or computation unit to evaluate the relative mobility as a function of the measured axial force with respect to the measured relative displacement.

Preferably, the computation unit is connected to a display unit to display the data processed by the computation unit, in particular the processing unit, for instance to display the relative mobility as a function of the measured axial force with respect to the measured relative displacement.

Alternatively, in another embodiment the computation unit is outside the housing of the device. The measuring module is connected to the computation unit to transfer the data outside the housing of the device. The processing of the measured displacements and the measured applied forces to evaluate the relative mobility as a function of the measured axial force with respect to the measured relative displacement is performed outside the housing.

Advantageously, the computation unit allows

- to calculate the indicators of interest;

- interact with the device, preferably with the measuring module (to determine Fmin and Fmax for example),

- superimpose the measurements of a same series to calculate the variability (standard deviation);

- export the data in appropriate format, for instance csv.

Preferably, the device further comprises a first metatarsal head M1 tip or support configured for being mounted on the first output shaft to be beard against the first metatarsal head M1, and a lesser metatarsal heads M2-M5 tip configured for being mounted on the second output shaft to be beard against the lesser metatarsal heads M2-M5. This allows to change the tips between two patients to comply with a sanitary protocol, for instance to avoid transmitting pathogens from one patient to another.

In a preferred embodiment the first metatarsal head M1 tip and the lesser metatarsal heads tip M2-M5 are reversibly mounted on respectively the first output shaft and the second output shaft, and wherein the first metatarsal head M1 tip and the lesser metatarsal heads tip M2-M5 are both compatible with the first output shaft and the second output shaft.

In otherwords, the tips are interchangeable: a same tip, namely the first metatarsal head M1 tip or the lesser metatarsal heads tip M2-M5, fits with the first output shaft and also with the second output shaft. When the device is first used with the right forefoot, the first metatarsal head M1 tip is on the first output shaft whereas the lesser metatarsal heads tip M2-M5 is on the second output shaft. Then, the tips are switched when the device is used with the left forefoot: the first metatarsal head M1 tip is now on the second output shaft whereas the lesser metatarsal heads tip M2-M5 is on the first output shaft. One device can be used with both forefeet by switching the tips.

Advantageously, the tips can be easily cleaned, notably the parts in contact with the skin of the patient. The tip can be chosen according to the morphology of each patient (shape of the tips can change while keeping the same principle of fixing with the output shaft).

Preferably, the transmission module is chosen among a gear transmission, a pivot plate pivotable around a pivot point, preferably a pivot plate pivotable around a pivot point. The transmission module can be any module or components assembly configured for equally distributing the axial force to two output shafts or at least two output shafts. Preferably, the transmission module supports the stresses or forces related to the measurements and reduce friction forces with several cylindrical bearings.

Advantageously, when the axial force is exerted on the transmission element and equally distributed to the first output shaft and to the second output shaft, the transmission element is configured for adopting a position or orientation depending the load exerted by the first output shaft and the second output shaft on the transmission element in response to the axial force exerted. In other words, the transmission element is configured to adopt a position or orientation depending on the relative mobility of the forefoot.

In a preferred embodiment, the transmission module comprises a pivot plate pivotable around a pivot point, the actuator being arranged for exerting an axial force on the pivot point of the pivot plate, the first output shaft being mounted on a first end of the pivot plate and the second output shaft being mounted on the opposed end of the pivot plate.

In an embodiment, when the axial force is exerted on the pivot plate and equally distributed to the first output shaft and to the second output shaft, the pivot plate is configured for adopting a position or orientation depending the load exerted by the first output shaft and the second output shaft on the pivot plate in response to the axial force exerted. In other words, the pivot plate is configured to adopt a position or orientation depending on the relative mobility of the forefoot.

For instance, the pivot plate can adopt either a first position or a second position,

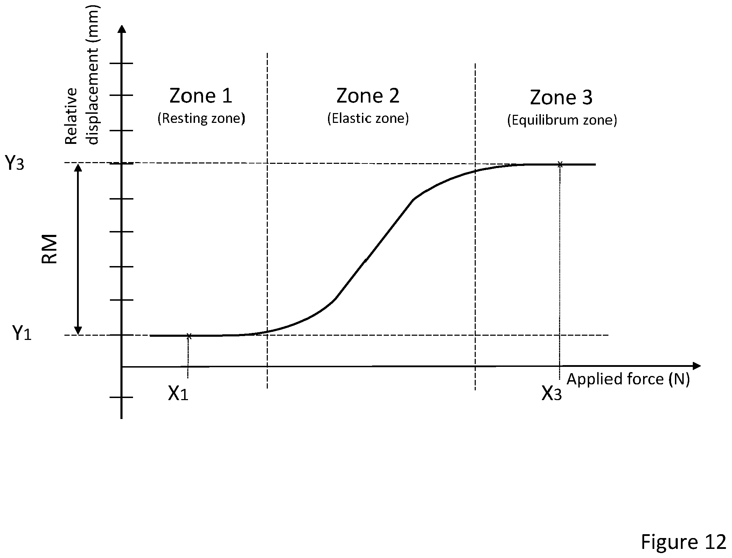

- a first position corresponding to a resting zone (zone 1 in figure 12) where the effort exerted by the first output shaft on the pivot plate is equal to the effort applied by the second output shaft on the pivot plate, and

- a second position corresponding to an equilibrium zone (zone 3 in figure 12) where the effort exerted by the first input shaft on the pivot plate is different from the effort applied by the second input shaft on the pivot plate.

In a preferred embodiment the displacement sensor is chosen among an angular sensor, a magnetic angular sensor, an optical encoder, a mechanical encoder, a graduated scale, a laser sensor, an electromagnetic sensor for instance a Linear Variable Differential Transformer LVDT, preferably a magnetic angular sensor. The displacement sensor can be any module or components assembly configured for measuring the relative displacement of the first metatarsal head M1 relative to the lesser metatarsal heads M2-M5 along said sagittal axis when beard respectively against the first output shaft and on the second output shaft.

In a preferred embodiment, the transmission module comprises a pivot plate and the displacement sensor is an angular sensor, said angular sensor being coupled to the pivot point of said pivot plate. The angular sensor is configured for measuring a pivot angle when the pivot plate is pivoting, for instance either clockwise (i.e. positive angle) or counter clockwise (i.e. negative angle). The measured pivot angle is used to compute the measured relative displacement by applying known trigonometry based mathematical formula.

For example, the pivot angle is measured between a first position and a second position of the pivot plate, the first position corresponding to a resting zone and the second position corresponding to an equilibrium zone (see figure 12).

In a preferred embodiment, the displacement sensor is a magnetic angular sensor. Magnetic angular sensors have many advantages such as being non-contact and miniature solution with high angular resolution and high sensitivity, linear characterization and low power consumption.

Preferably, the force sensor is chosen among load cell, compression load cell, spring dynamometer.

In a preferred embodiment, the force sensor is a load cell preferably compression load cell. Such sensors have many advantages such as digital format, good resolution and sensitivity, small footprint, low power consumption and linear characterization.

In an embodiment, the device comprises a feedback module. For instance the feedback module is configured for providing a feedback to the practitioner. Advantageously, the feedback allows the practitioner to evaluate, preferably measure, the force applied on the forefoot, notably to evaluate the increase or decrease of the applied force.

Preferably, the feedback is haptic feedback, visual feedback and/or audible feedback, preferably visual feedback and/or audible feedback.

In a preferred embodiment, the feedback module comprises a visual display to provide visual feedback.

In an embodiment, the feedback module comprises a microphone (or a speaker or a miniature speaker) to emit a sound signal.

Preferably, the feedback given is adaptable to the force applied.

In an embodiment, the present invention, preferably the system, comprises a graduated scale configured for calibrating the distribution of the force between the first output shaft and the second output shaft. Alternatively, the graduated scale is configured for calibrating the displacement of the first output shaft and second output shaft, for instance the relative displacement of, preferably by using predetermined relative displacement.

The present invention also concerns a system for evaluating the relative mobility of a forefoot of a patient, the system comprising

a device according to the present invention;

an orthosis configured for maintaining the forefoot of the patient in position suitable for measuring the relative mobility between the first metatarsal head and the lesser metatarsal heads of a forefoot of a patient;

a positioning module configured for reversibly fastening the device to the orthosis.

In a preferred embodiment, the orthosis is equipped with a sole configured for receiving the foot of the patient. Preferably the sole is molded to the foot of the patient to maintain the feet in a position allowing the forefoot analysis.

The positioning module acts as in interface between the device and the orthosis. The positioning module allow to control the relative position of the orthosis with respect to the device taking the measurements. When the forefoot of the patient is received in the orthosis, the positioning module allows to maintain the feet in a suitable position relative to the device to perform the evaluation of the relative mobility of the forefoot of the patient with the device according to the present invention.

In a preferred embodiment the positioning module comprises reversible fastening means for fastening the positioning module to the orthosis. Preferably, the fastening means also allow to fasten in a reversible manner the positioning module with the device.

Preferably, the reversible fastening means fastening the device to the positioning module are configured for controlling the translation of the device with respect to the positioning module. The translation axis allows to adjust the position of the device along the metatarsal head of the forefoot.

Preferably, the reversible fastening means fastening the orthosis to the positioning module are configured for controlling the translation of the orthosis with respect to the positioning module. The translation axis allows to adjust the position of the device on the metatarsal head of the forefoot.

Advantageously, the positioning module allows the device to be adjusted to each foot along three axes, preferably thee translation axes, meaning in three dimensions (x,y,z).

The positioning module has two main functions: fixing the position of the device relative to the orthosis along two translation axes and secondly allowing the displacement along a third translation axis to bear the device on the forefoot by and exert the axial force on said forefoot.

In other words, firstly, the positioning module allows to immobilize the position of the orthosis and device along two perpendicular translation axes :

-  a first translation axis extending along the foot sole once the foot is maintained in the orthosis, and

-  a second translation axis perpendicular to said first translation axis to position the device opposite (i.e. under) the metatarsal heads of the forefoot.

Secondly, the device remains displaceable along a third translation axis extending along the sagittal axis to position the first output shaft and the second output shaft respectively opposite the first metatarsal head M1 and the lesser metatarsal heads M2-M5 to exert the axial force on the metatarsal heads and measure the relative displacement.

Preferably, the reversible fastening means comprises a pin and a slider arranged for receiving said pin, the pin being arranged sliding in the slider in a translation motion. Alternatively, the fastening means can comprise knurled screw, worm gear and pinion.

In a preferred embodiment, the device comprises a connection module for connecting said device with an external module. The connection module allows a wireless transfer of the measurements taken by the device, for instance the applied force or the displacement measurements. For instance, the connection module comprises a wireless module for connection in a wireless manner the device with an external module. For instance, the external module is a computation unit configured for processing the measurements acquired by the measuring module to evaluate the relative mobility as a function of the measured axial force with respect to the measured relative displacement.

Alternatively, the external module is a display module to display the data processed by the computation module embedded in the device, meaning in the housing of the device, to evaluate the relative mobility as a function of the measured axial force with respect to the measured relative displacement.

The present invention also relates to a method, preferably computer implemented method, for computing a relative mobility score of a forefoot of a patient, the method comprising

    i) providing a set of data $(x_i, y_i)$ where

        $x_i$ represents a measured applied force along the sagittal axis and equally distributed on the first metatarsal head M1 of the forefoot and on the lesser metatarsal heads M2-M5 of the forefoot, and
        $y_i$ represents a relative displacement of the first metatarsal head M1 relative to the lesser metatarsal heads M2-M5 along said sagittal axis upon application of the corresponding measured $x_i$ applied force,

    ii) representing the relative displacement of the first metatarsal head M1 relative to the lesser metatarsal heads M2-M5 $(y_i)$ as a function of the measured applied force $(x_i)$ for each $(x_i, y_i)$;

    iii) determining Xmin and Xmax as respectively the minimum measured force applied and the maximum measured force applied, and
determining Ymin and Ymax as respectively the minimum measured relative displacement for Xmin and the maximum measured relative displacement at Xmax,

    iv) computing the relative mobility score RM as

$$RM = Ymax - Ymin.$$

Advantageously, the relative mobility score RM is a parameter allowing to determine the mobility of the first ray which plays an important role in various pathologies of the foot (e.g. Hallux valgus). An indication of the first ray mobility assists the practitioner to make the most appropriate therapeutic decision for the patient (e.g. the type of surgical intervention in case of an operation of the forefoot)

Preferably,

    Xmin is comprised between about 0 N and about 30 N, preferably between about 0 N and about 20 N, more preferably between about 5 N and 20 N, more preferably between about 5 N and 15 N, more preferably between about 5 N and 10 Nm
    and/or

    Xmax is comprised between about 60 N and about 120 N, preferably between about 80 N and about 110 N, more preferably between about 80 N and 100 N, more preferably between about 80 N and 90 N

Preferably, Xmin is between about 5N and about 10N to remove or limit the background noise such as involuntary motion that might occur below or equal to about 5 N.

Preferably, Xmax is comprised between about 90 N and 100 N to ensure to have reached an equilibrium zone (zone 3 - see figure 12).

Preferably, Xmax depends on the position of the patient. For instance, Xmax can be higher than 120 N when the patient is in a standing position.

Preferably, Ymin and Ymax vary and depend on the forefoot of the patient.

The invention further relates to a method, preferably computer implemented method, for computing a differential stiffness score of a forefoot of a patient, the method comprising

    i) providing a set of data $(x_i, y_i)$ where

        $x_i$ represents a measured applied force along the sagittal axis and equally distributed on the first metatarsal head M1 of the forefoot and on the lesser metatarsal heads M2-M5 of the forefoot, and

yi represents a relative displacement of the first metatarsal head M1 relative to the lesser metatarsal heads M2-M5 along said sagittal axis upon application of the corresponding measured xi applied force,

ii) representing the relative displacement of the first metatarsal head M1 relative to the lesser metatarsal heads M2-M5 (yi) as a function of the measured applied force (xi) for each (xi,yi);

iii) determining Xmin and Xmax as respectively the minimum measured force applied and the maximum measured force applied, and
determining Ymin and Ymax as respectively the minimum measured relative displacement for Xmin and the maximum measured relative displacement at Xmax,

iv) calculating the local derivatives at any points of the curve representing the relative displacement as a function of the force applied between Xmin, Xmax and Ymin, Ymax.

v) computing the differential stiffness score as the maximum value of the calculated local derivatives.

Advantageously, the differential stiffness score is a parameter allowing to determine an indication of the elastic properties of the first ray of the foot.

In another aspect, the invention relates to a method for evaluating the relative mobility of a forefoot of a patient, the method comprising

i) applying an axial force on the forefoot of the patient along the sagittal axis, said axial force being equally distributed between the first metatarsal head M1 relative to the lesser metatarsal heads M2-M5,

ii) measuring a relative displacement of the first metatarsal head M1 relative to the lesser metatarsal heads M2-M5 while applying said axial force;

iii) correlating the measured displacements with the applied axial forces to determine the evolution of the relative displacement of the first metatarsal head M1 relative to the lesser metatarsal heads M2-M5 as a function of the force applied;

The particular advantages of the methods are similar to the ones of the device of the invention and will thus not be repeated here.

As used herein, the word "means" (singular or plural) preceded or followed by a function can be replaced by the word "unit" or" module". For instance "fastening means" can be replaced by "fastening unit" or "fastening module".

As used herein, the term "about" applies to numeric values or ranges of numeric values and refers to a range of numbers that one of skill in the art would consider equivalent to the recited values, i.e. plus or minus ten percent. For example, "about 10 cm" refers to 10 cm +/- 10%, i.e. 9 cm to 11 cm.

The embodiments describe for the device also apply to the methods or the system according to the present invention mutatis mutandis.

**Brief description of the drawings**

Further particular advantages and features of the invention will become more apparent from the following non-limitative description of at least one embodiment of the invention which will refer to the accompanying drawings, wherein

- Figures 1 to 7 represent the device and the system according to the present invention;
- Figures 8 and 9 represent the positioning module of the system according to the present invention;
- Figures 10 and 11 represent the equal force distribution on the applied force on the first output shaft and on the second output shaft; figure 10 : F : applied force (N), F1 = F2 : the applied force (F) equally distributed in two forces; k1 and k2 : Stiffness coefficients exerted on the metatarsal supports by the first ray and rays two to five(M1 and M2M5))
- Figure 12 and 13 illustrate the relative mobility score computed by the method according to the present invention;

**Detailed description of the invention**

The present detailed description is intended to illustrate the invention in a non-limitative manner since any feature of an embodiment may be combined with any other feature of a different embodiment in an advantageous manner.

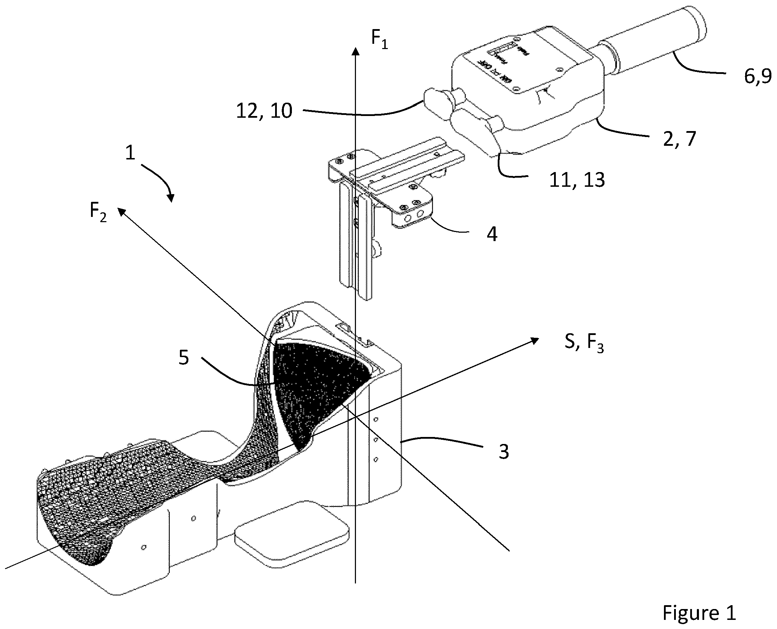

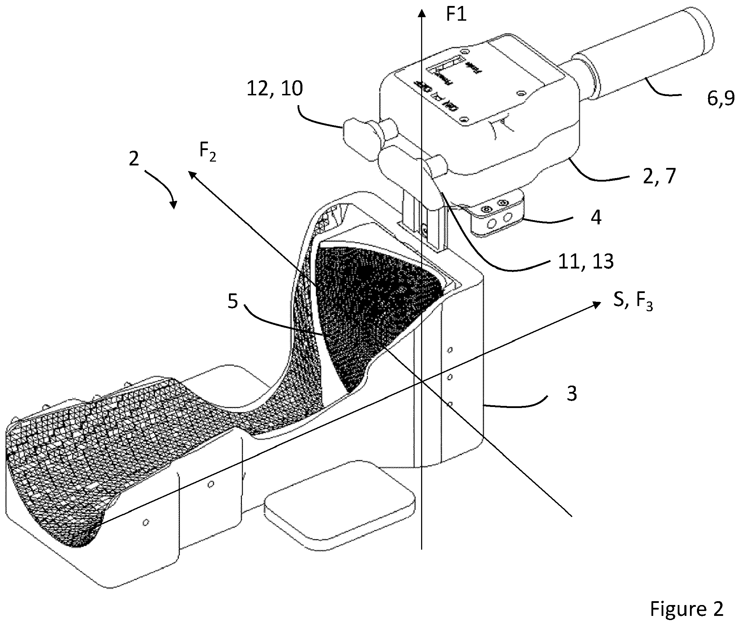

Figures 1 to 4 represent a general overview of an embodiment of the system 1 for evaluating the relative mobility of a forefoot of a patient according to the present invention. However, the invention is not limited to the example shown in the figures and detailed below.

The system 1 comprises a device 2 mounted on an orthosis 3 via a positioning module 4. The device 2, the orthosis 3 and the positioning module 4 are attachable and detachable one another, in other words reversibly attached, the system 1 is modular. This facilitates the transport and/or the storing of the system 1 since the different components can be disassembled before and store in a container suitable for the disassembled system (but unsuitable for the assembled system 1).

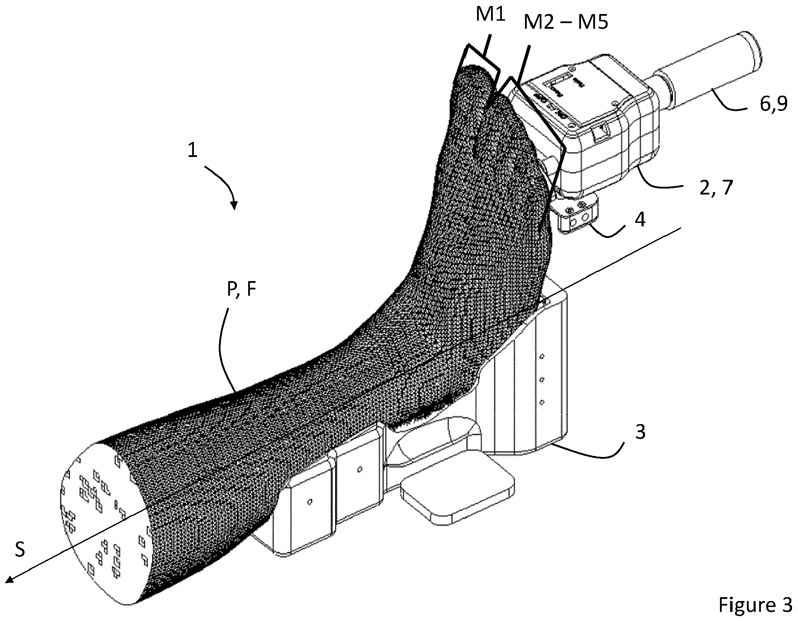

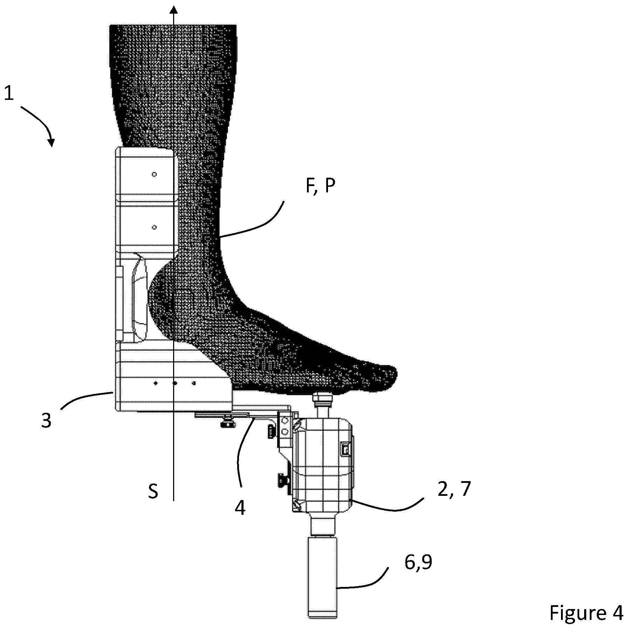

As shown in figures 3 and 4, the orthosis 3 is configured for receiving the foot F of the patient P. the patient P can be either in a lying position as shown in figure 3 or in a sanding position as shown in figure 4. In other embodiments (not shown), the patient can be in any positions between the standing position shown in figure 4 and the lying position shown in figure 3.

In the illustrated embodiment, the orthosis 3 is equipped with a sole 5 configured for receiving the foot of the patient. preferably, a right foot sole is mounted when a right foot is received in the orthosis 3 and a left foot sole is mounted when a left foot is mounted in the orthosis. The orthosis can therefore be used with both feet. In the present example, the orthosis 3 is equipped with a right foot sole.

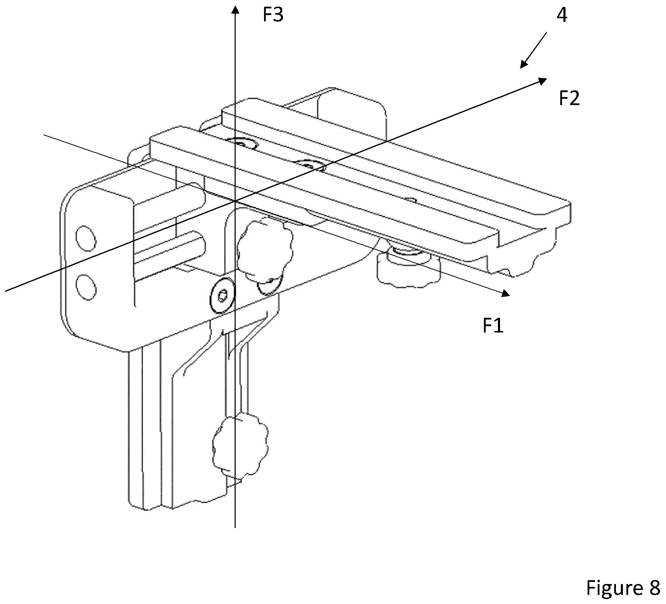

As shown in figures 1,2 and 8,9, in the present example the positioning module allows the device to be adjusted to each foot along three translation axes in three dimensions (x,y,z).

The device, the orthosis and the device are assembled as followed :

- fixing the position of the device relative to the orthosis along two translation axes F1 and F2 ;
- allowing the displacement along a third translation axis F3 to bear the device on the forefoot by and exert the axial force on said forefoot.

In other words, firstly, the positioning module immobilizes the position of the orthosis and device along two perpendicular translation axes :

- a first translation axis F1 extending along the foot sole once the foot is maintained in the orthosis, and

- a second translation axis F2 perpendicular to said first translation axis to position the device opposite (i.e. under) the metatarsal heads of the forefoot.

Secondly, the device remains displaceable along a third translation axis extending F3 along the sagittal axis to position the first output shaft and the second output shaft respectively opposite the first metatarsal head M1 and the lesser metatarsal heads M2-M5 to exert the axial force on the metatarsal heads and measure the relative displacement.

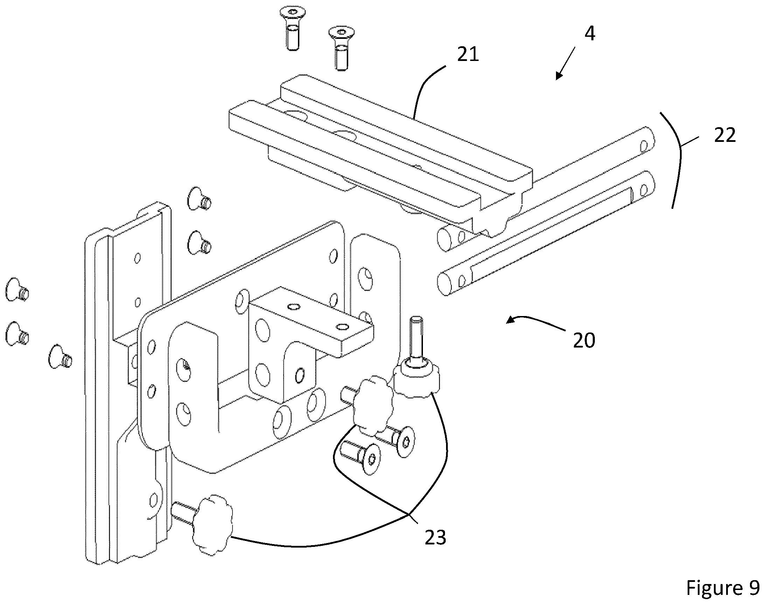

The positioning module 4 comprises fastening means for adjusting the relative position of the device relative to the orthosis 3 and to the forefoot F received in said orthosis 3. As shown in figure 9, in the present example the fastening means comprises a sliding component assembly 20, notably sliding supports 21 sliding along sliding pins 22 and stoppers to lock the positions of said sliding supports 21.

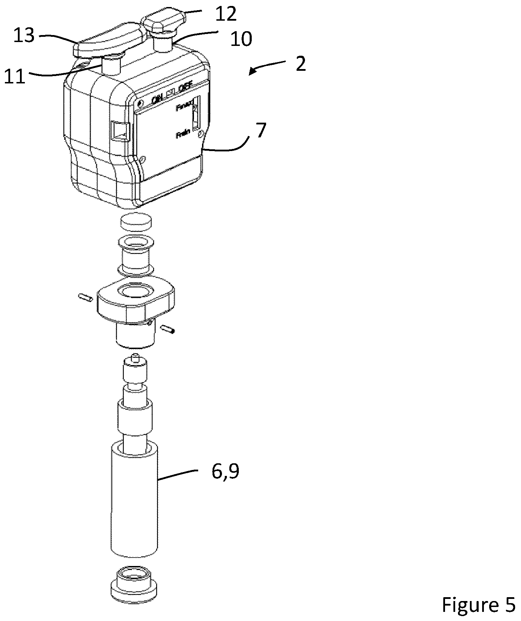

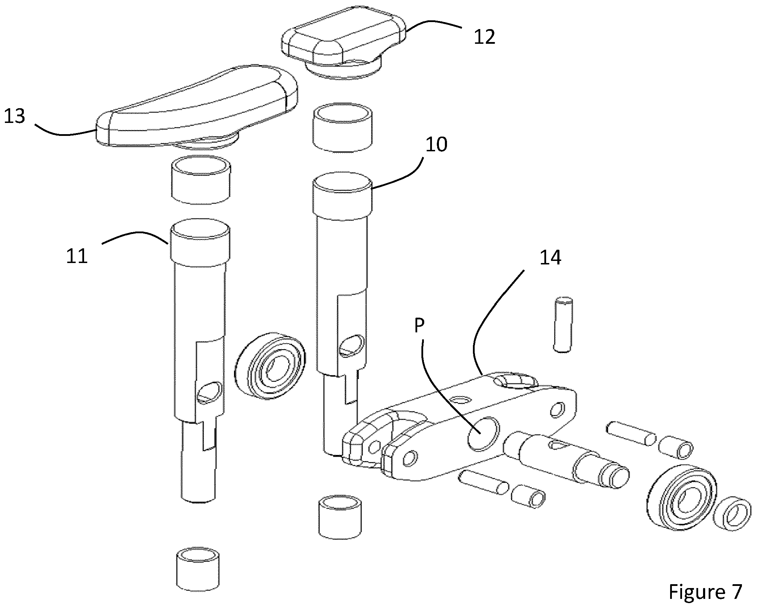

Figures 5 to 7 illustrated in a detailed manner the device 2 according to the present invention. The device 2 comprises a housing 7 hosting the various components of the device 2.

The device 2 further comprises a transmission element 8, and actuator 9, and two outputs shafts namely a first output shaft 10 and a second output shaft 11. The transmission element 8 is configured for equally distributing an axial force provided by the actuator 9 along the sagittal axis S of the patient P to the first output shaft 10 and to the second output shaft 11. The first shaft 10 is configured for bearing against the first metatarsal head M1 of the forefoot whereas the second shaft being configured for bearing against the lesser metatarsal heads M2-M5 of the forefoot. The device further comprises a first metatarsal head M1 tip 12 and a lesser metatarsal heads tip M2-M5 13 reversibly mounted on respectively the first output shaft 10 and the second output shaft 11.

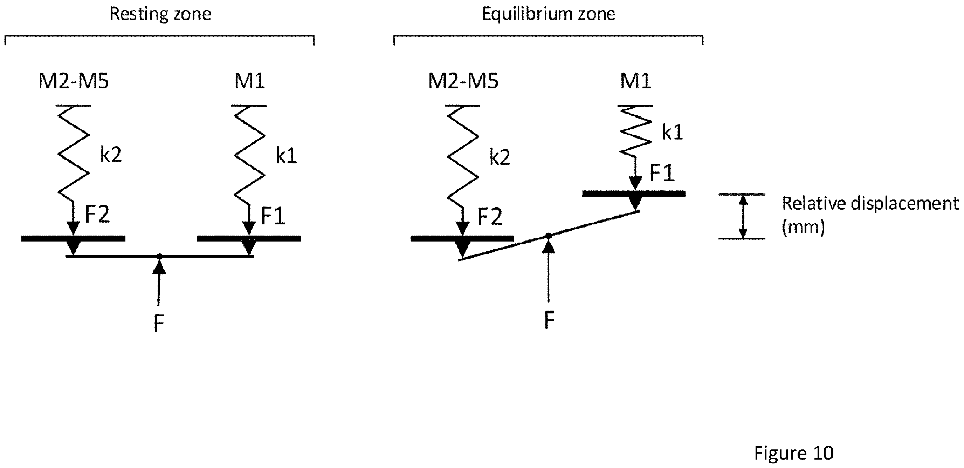

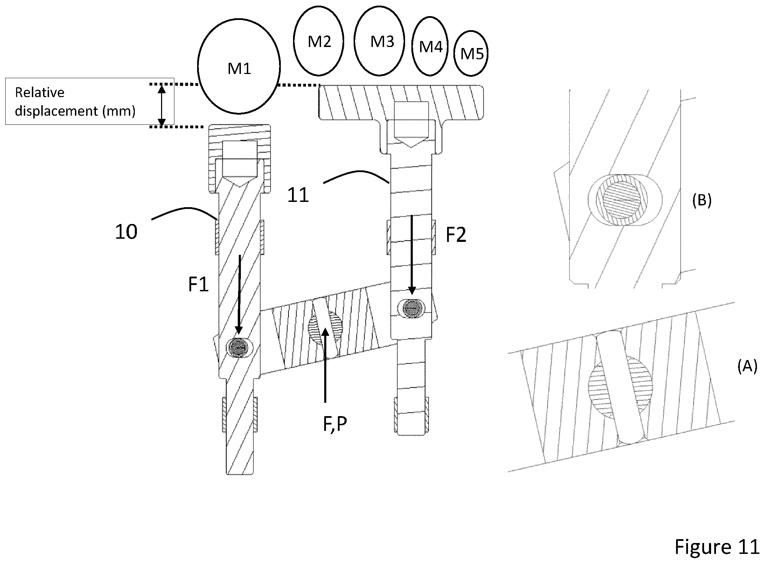

In other words, the force exerted by the actuator 9 is transmitted to the first output shaft 10 and to the second output shaft 11 via the transmission element 8. The transmission element 8 is configured for controlling the force exerted by the actuator 9 to ensure an equal distribution of said force between the first output shaft 10 and the second output shaft 11. This is illustrated in figures 10,11. The force applied F from the actuator 9 is transmitted by the transmission element 8 to the first output shaft 10 and to the second output shaft 11. A force F1 from the first output shaft 10 is applied on the first metatarsal head M1 and a force F2 from the second output shaft 11 is applied on the lesser metatarsal heads M2-M5, F1 being equal to F2.

In the present example, the device 2 is configured for being manually actuated and the actuator 9 comprises the hand 6 outside the housing and fixed to the housing 7. A user, handing the hand 6 as actuator 9, provides or exerts the axial force applied on the transmission element 8.

The transmission element 8 can be any module or components assembly configured for equally distributing the axial force to two output shafts or at least two output shafts. In the present example, the transmission element 8 comprises a pivot plate 14 pivotable around a pivot point P. The pivot plate acts as a balance or a swing tilting on one end or on the other end around the pivot point depending on the effort applied on the respective ends. The actuator 9 is arranged for exerting the axial force on the pivot point P of the pivot plate P.

The first output shaft 10 is mounted on a first end of the pivot plate 14, and the second output shaft 11 is mounted on the opposed end of the pivot plate 14.

As shown in figures 10,11, when the axial force is exerted on the pivot plate 14 and equally distributed to the first output shaft 10 and to the second output shaft 11, the pivot plate 14 is configured for adopting a first position or a second position,

- a first position P1 corresponding resting zone (zone 1 in figure 12- ) where the effort exerted by the first output shaft 10 on the pivot plate 14 is equal to the effort applied by the second output shaft 11 on the pivot plate 14, and

- a second position P2 corresponding to an equilibrium zone (zone 3 in figure 12) where the effort exerted by the first input shaft 10 on the pivot plate 14 is different from the effort applied by the second input shaft 11 on the pivot plate 14; either F2 is superior to F1 (as shown in figures 10,11) or F1 is superior to F2 (not shown).

When the pivot plate is in position P2, a relative displacement between the first metatarsal head M1 and the lesser metatarsal heads can be measured by a measuring module 15.

The measuring module 15 comprises

a force sensor 16 configured for measuring the axial force exerted by the actuator 9, and
a displacement sensor 17 configured for measuring the relative displacement of the first metatarsal head M1 relative to the lesser metatarsal heads M2-M5 along said sagittal axis when beard respectively against the first output shaft 10 and on the second output shaft 11.

In the present embodiment, the force sensor 16 is a compressible cell load 18. The displacement sensor is a magnetic angular sensor 19 (inside the housing 7- not shown in figures).

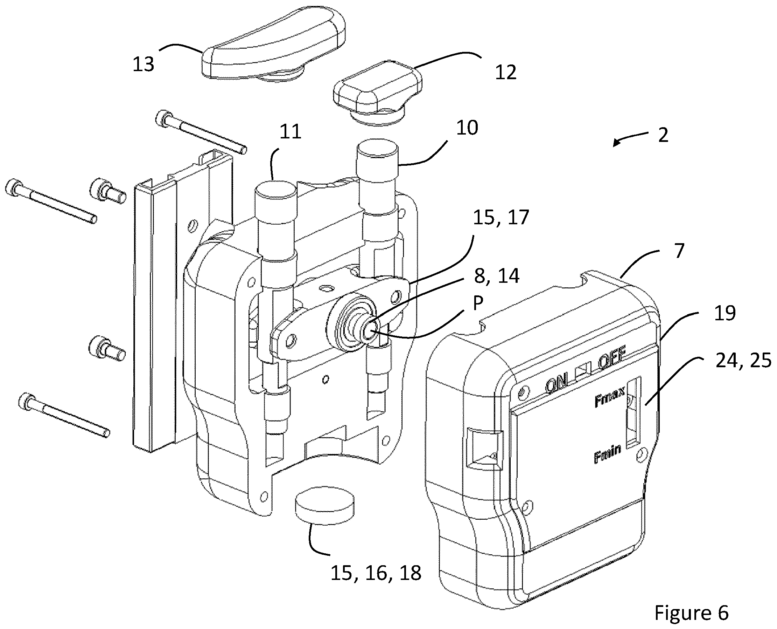

As shown in figure 6, the device further comprises a feedback module 24 to help the practitioner to evaluate and measure the force applied on the forefoot. In the present example, the feedback module is a visual display meaning a graduated scale 25 between Fmin and Fmax to evaluate the axial force applied by the partitioner on the actuator 9.

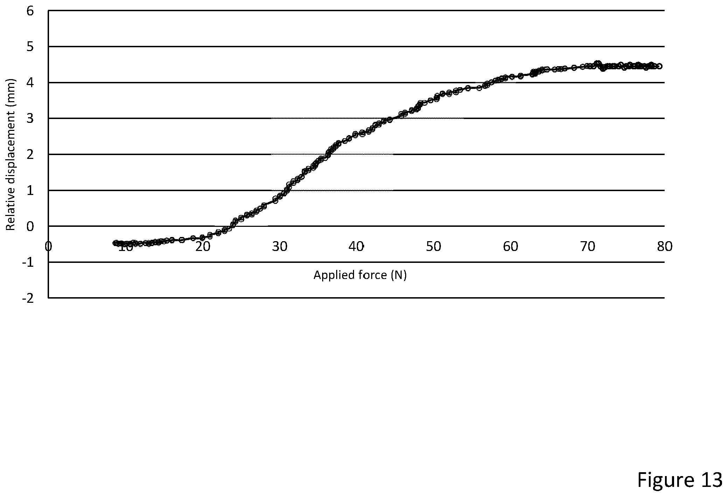

The measuring module 15 is configured for correlating the displacement measurements made by the magnetic angular sensor in this example with the measured axial forces made by the compression cell load in this example to evaluate the relative mobility of the forefoot of the patient. In this example, the force sensor 16 and the relative displacement sensor 17 operate simultaneously so that each measured axial force (xi) is associated with a measured relative displacement (yi). Therefore, it is possible to determine a relative mobility score RM corresponding to the displacement of the first metatarsal head M1 relative to the lesser metatarsal heads M2-M5 as a function of the axial force applied. This is shown in figures 12,13.

Figure 12 represents a theorical scheme or curve of the displacement of the first metatarsal head M1 relative to the lesser metatarsal heads M2-M5 (on the y axis) as a function of the axial force applied (on the x axis).

The theorical curve comprises three zones or areas

- zone 1: resting zone corresponding to a plateau, during which the foot remains at a resting position. In other words, the applied force is incapable of causing a displacement between the first metatarsal head M1 and the lesser metatarsal heads M2-M5;

- zone 2: elastic zone corresponding to a dynamic displacement between the first metatarsal head M1 and the lesser metatarsal heads M2-M5 as a function of the applied force where the relative displacement increases with the applied force.

- zone 3 : equilibrium zone corresponding to a portion where the curve (relative displacement Yi as a function of the applied force xi) depicts a second plateau, during which the maximum relative displacement has been obtained. No additional displacement will be detected when the applied force increases.

In the present case shown in figure 12, Fmin corresponds to X1 and Fmax corresponds to X3 while Ymin corresponds to Y1 and Y3 corresponds to Ymax.

Figure 13 is an example of a calculate RM for a patient:

- F min = 10N

- Fmax = 80N

- Ymin = -0.48mm

- Ymax = 4.46mm

- RM = 4.46 - (-0.048) = 4.94mm

While the embodiments have been described in conjunction with a number of embodiments, it is evident that many alternatives, modifications and variations would be or are apparent to those of ordinary skill in the applicable arts. Accordingly, this disclosure is intended to embrace all such alternatives, modifications, equivalents and variations that are within the scope of this disclosure. This for example particularly the case regarding the different apparatuses which can be used.

**REFERENCE NUMBERS**

| 1 | System according to an embodiment of the present invention |
| 2 | Device according to an embodiment of the present invention |
| 3 | Orthosis |
| 4 | Positioning module |
| F | Foot |
| P | Patient |
| 5 | Sole of the orthosis |
| 6 | Hand |
| 7 | Housing |
| 8 | Transmission element |
| 9 | Actuator |
| 10 | First output shaft |
| 11 | Second output shaft |
| S | Sagittal axis, in particular dorsal direction |

12 First metatarsal head M1 tip
13 Lesser metatarsal heads tip M2-M5
14 Pivot plate
P Pivot point
15 Measuring module
16 Force sensor
17 Displacement sensor
18 Compression cell load
19 Magnetic angular sensor
20 Sliding assembly
21 Sliding support
22 Sliding pin
23 Stopper
24 Feedback module
25 Visual display

**Claims**

1. Device (2) for evaluating the relative mobility of a forefoot (F) of a patient (P), the device (2) comprising an actuator (9), a transmission element (8) and two output shafts namely a first output shaft (10) and a second output shaft (11),

the transmission element (8) being configured for equally distributing an axial force provided by the actuator (9) along the sagittal axis (S) of the patient (P) to the first output shaft (10) and to the second output shaft (11), the first output shaft (10) being configured for bearing against the first metatarsal head M1 of the forefoot whereas the second output shaft being configured for bearing against the lesser metatarsal heads M2-M5 of the forefoot,
**characterized in that** the device (2) further comprises a measuring module (15) comprising a force sensor (16) configured for measuring the axial force exerted by the actuator (9), and
a displacement sensor (17) configured for measuring the relative displacement of the first metatarsal head M1 relative to the lesser metatarsal heads M2-M5 along said sagittal axis (S) when beard respectively against the first output shaft (10) and on the second output shaft (11), the measuring module (15) being configured for correlating said displacement measurements with said measured axial forces so as to evaluate the relative mobility of the forefoot (F) of the patient (P).

2. Device (2) according to claim 1, wherein the measuring module (15) is configured for measuring simultaneously the force applied and the relative displacement.

3. Device (2) according to claim 1 or 2, wherein the device (2) is configured for evaluating the relative mobility of the patient (P) when the patient (P) is in a standing position and in a lying position and any positions between a standing position and a lying position.

4. Device (2) according to any one of claims 1 to 3, wherein the actuator (9) is configured for being actuated manually by a user of the device providing the axial force, preferably the actuator (9) comprising a hand (6) to be handled by the user.

5. Device (2) according to any one of claims 1 to 3, wherein the actuator (9) is configured for being actuated by driving element commanding the actuation, preferably a motor, to provide the axial force.

6. Device (2) according to any one of claims 1 to 5, wherein the device (2) further comprises a computation unit configured for processing the measurements acquired by the measuring module (15) to evaluate the relative mobility as a function of the measured axial force with respect to the measured relative displacement.

7. Device (2) according to any one of claims 1 to 6, wherein the device (2) further comprises a first metatarsal head M1 tip (12) configured for being mounted on the first output shaft (10) to be beard against the first metatarsal head M1, and a lesser metatarsal heads M2-M5 tip (13) configured for being mounted on the second output shaft (11) to be beard against the lesser metatarsal heads M2-M5.

8. Device (2) according to the preceding claim, wherein the first metatarsal head M1 tip (12) and the lesser metatarsal heads tip M2-M5 (13) are reversibly mounted on respectively the first output shaft (10) and the second output shaft (11), and wherein the first metatarsal head M1 tip (12) and the lesser metatarsal heads tip M2-M5 (13) are both compatible with the first output shaft (10) and the second output shaft (11).

9. Device (2) according to any one of claims 1 to 8, wherein the transmission module (8) is chosen among a gear transmission, a pivot plate (14) pivotable around a pivot point (P), preferably a pivot plate (14) pivotable around a pivot point (P).

10. Device (2) according to any one of claims 1 to 9, wherein the displacement sensor (17) is chosen among an angular sensor, a magnetic angular sensor (19), an optical encoder, a mechanical encoder, a graduated scale, a laser sensor, an electromagnetic sensor for instance a Linear Variable Differential Transformer LVDT, preferably a magnetic angular sensor (19).

**11.** Device (2) according to any one of claims 1 to 10, wherein the force sensor (16) is chosen among cell load, compression cell load (18), spring dynamometer.

**12.** System (1) for evaluating the relative mobility of a forefoot (F) of a patient (P), the system (1) comprising

- a device (2) according to any one of claims 1 to 11
- an orthosis (3) configured for maintaining the forefoot of the patient in position suitable for measuring the relative mobility between the first metatarsal head and the lesser metatarsal heads of a forefoot of a patient;
- a positioning module (4) configured for reversibly fastening the device (2) to the orthosis (3).

**13.** Method, preferably computer implemented method, for computing a relative mobility score of a forefoot (F) of a patient (P), the method comprising

i) providing a set of data (xi,yi) where

xi represents a measured applied force along the sagittal axis and equally distributed on the first metatarsal head M1 of the forefoot and on the lesser metatarsal heads M2-M5 of the forefoot, and
yi represents a relative displacement of the first metatarsal head M1 relative to the lesser metatarsal heads M2-M5 along said sagittal axis upon application of the corresponding measured xi applied force,

ii) representing the relative displacement of the first metatarsal head M1 relative to the lesser metatarsal heads M2-M5 (yi) as a function of the measured applied force (xi) for each (xi,yi);
iii) determining Xmin and Xmax as respectively the minimum measured force applied and the maximum measured force applied, and determining Ymin and Ymax as respectively the minimum measured relative displacement for Xmin and the maximum measured relative displacement at Xmax,
iv) computing the relative mobility score RM as

$$RM = Ymax - Ymin$$

**14.** Method according to the preceding claim, wherein

Xmin is comprised between about 0 N and about 30 N, preferably between about 0 N and about 20 N, more preferably between about 5 N and 20 N, more preferably between about 5 N and 15 N, more preferably between about 5 N and 10 N,
and/or
Xmax is comprised between about 60 N and about 120 N, preferably between about 80 N and about 110 N, more preferably between about 80 N and 100 N, more preferably between about 80 N and 90 N.

**15.** Method, preferably computer implemented method, for computing a differential stiffness score of a forefoot (F) of a patient (P), the method comprising

i) providing a set of data (xi,yi) where

xi represents a measured applied force along the sagittal axis and equally distributed on the first metatarsal head M1 of the forefoot and on the lesser metatarsal heads M2-M5 of the forefoot, and
yi represents a relative displacement of the first metatarsal head M1 relative to the lesser metatarsal heads M2-M5 along said sagittal axis upon application of the corresponding measured xi applied force,

ii) representing the relative displacement of the first metatarsal head M1 relative to the lesser metatarsal heads M2-M5 (yi) as a function of the measured applied force (xi) for each (xi,yi);
iii) determining Xmin and Xmax as respectively the minimum measured force applied and the maximum measured force applied, and determining Ymin and Ymax as respectively the minimum measured relative displacement for Xmin and the maximum measured relative displacement at Xmax,
iv) calculating the local derivatives at any points of the curve representing the relative displacement as a function of the force applied between Xmin, Xmax and Ymin, Ymax.
v) computing the differential stiffness score as the maximum value of the calculated local derivatives.

Figure 1

Figure 2

Figure 3

Figure 4

Figure 5

EP 4 272 640 A1

Figure 6

Figure 7

Figure 8

Figure 9

Figure 10

Figure 11

Relative displacement (mm)

Zone 1
(Resting zone)

Zone 2
(Elastic zone)

Zone 3
(Equilibrum zone)

$Y_3$

$Y_1$

RM

$X_1$

$X_3$

Applied force (N)

Figure 12

Figure 13

Europäisches
Patentamt

European
Patent Office

Office européen
des brevets

# EUROPEAN SEARCH REPORT

**Application Number**

EP 22 17 2071

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2017/171660 A1 (UNIV NANYANG TECH [SG]) 5 October 2017 (2017-10-05) * figures 3,5,9 * * page 2, lines 8-15 * * page 4, lines 25-31 * * page 5, lines 4-15 * * page 6, lines 8,22-23,35 * * page 7, lines 17-20 * * page 8, lines 28-30 * * page 9, lines 2-3,33-36 * * page 10, lines 17-19 * ----- | 1-15 | INV. A61B5/11 A61B5/00 |
| A | GLASOE WARD MYLO ET AL: "Measuring first ray mobility with a new device", ARCHIVES OF PHYSICAL MEDICINE AND REHABILITATION, vol. 80, no. 1, 1 January 1999 (1999-01-01), pages 122-124, XP055813238, AMSTERDAM, NL ISSN: 0003-9993, DOI: 10.1016/S0003-9993(99)90320-9 * figures 1-3 * ----- | 1-15 | |

| | |
|---|---|
| | TECHNICAL FIELDS SEARCHED (IPC) |
| | A61B |

The present search report has been drawn up for all claims

| Place of search | Date of completion of the search | Examiner |
|---|---|---|
| The Hague | 10 October 2022 | Chau, Thoi Dai |

EPO FORM 1503 03.82 (P04C01)

## ANNEX TO THE EUROPEAN SEARCH REPORT
## ON EUROPEAN PATENT APPLICATION NO.

EP 22 17 2071

This annex lists the patent family members relating to the patent documents cited in the above-mentioned European search report.
The members are as contained in the European Patent Office EDP file on
The European Patent Office is in no way liable for these particulars which are merely given for the purpose of information.

10-10-2022

| Patent document cited in search report | Publication date | Patent family member(s) | Publication date |
|---|---|---|---|
| WO 2017171660 A1 | 05-10-2017 | NONE | |

For more details about this annex : see Official Journal of the European Patent Office, No. 12/82

EPO FORM P0459